# EUROPEAN PATENT APPLICATION

(11) **EP 1 967 131 A1**
(43) Date of publication of application: **10.09.2008**
(21) Application number: 08001066.3
(22) Date of filing: 21.01.2008
(51) Int. Cl.: A61B 1/01

(54) **Method of working end portion of tube for medical instrument**

(30) Priority: 07.03.2007 JP 2007057697
(71) Applicant: Fujinon Corporation, Saitama-shi, Saitama (JP)
(72) Inventor: Fujikura, Tetsuya, Saitama-shi Saitama (JP)
(74) Representative: Höhfeld, Jochen

(57) **Abstract**

It is an object of the invention to provide a method of working an end portion of a tube for a medical instrument capable of being worked easily in a short period of time and capable of providing a smooth end portion of an outer surface thereof.
According to a method of working an end portion of a tube for a medical instrument, first, a core member is inserted into a thermoplastic tube and arranged at a front end of the tube, a thermally contracting tube is covered to cover the front end of the tube, and heat is applied to the thermally contracting tube to contract at a position of the front end to thereby deform the front end of the tube by a contracting force of the thermally contracting tube and the heat from the thermally contracting tube.

## Description

### BACKGROUND

### 1. Technical Field

The present invention relates to a method of working an end portion of a tube for a medical instrument, particularly relates to a method of working an end portion of an insertion assisting piece used in inserting an endoscope.

### 2. Related Art

As a guide in inserting an inserting portion of an endoscope into the body, an insertion assisting piece (also referred to as overtube or sliding tube) is generally used. An insertion assisting piece is a tube inserted with an inserting portion of an endoscope. The insertion assisting piece is inserted into the body in a state of being inserted with the inserting portion of the endoscope and is arranged at a flexing portion of the digestive tract. Thereby, the inserting portion of the endoscope is guided by the insertion assisting piece. Therefore, extraneous flexing or bending of the inserting portion can be prevented and the inserting portion can be easily inserted to a deep portion in the body.

Meanwhile, according to the insertion assisting piece, a front end of the insertion assisting piece is a converging shape by contracting the front end of the insertion assisting piece (refer to, for example, JP-A-2001-340462). This is for preventing the body wall (for example, the intestinal wall) from being entrapped into the insertion assisting piece, orpreventing a lubricant supplied to between the insertion assisting piece and the inserting portion of the endoscope from being leaked out by reducing a gap between the front end of the insertion assisting piece and the inserting portion of the endoscope.

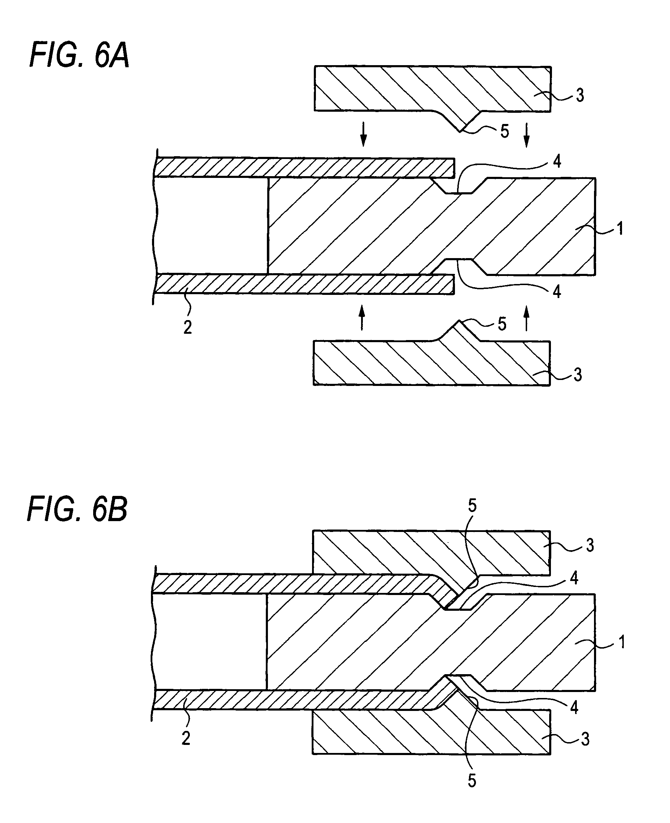

The insertion assisting piece having a converging shape is fabricated by thermally deforming a thermoplastic tube by a die. Specifically, as shown in Fig. 6A, after inserting a core 1 in a circular column shape into a thermoplastic tube 2 (insertion assisting piece), as shown in Fig. 6B, a halved pair of dies 3, 3 are pressed to the tube 2 from an outer side thereof. The core 1 is formed with a groove 4 over one periphery, and a front end of the thermoplastic tube 2 is arranged at a position of the groove 4. Further, the pair of dies 3, 3 forms a cylindrical body by being combined, and inner peripheral faces thereof are formed with projected streak portions 5 brought into the groove 4 of the core 1. Further, after squeezing the front end of the thermoplastic tube 2 by the core 1 and the pair of dies 3, 3, by heating the pair of dies 3, 3, the front end of the thermoplastic tube 2 is thermally deformed to be worked into the converging shape.

However, when worked by the related art method, there poses a problem that a portion of the thermoplastic tube other than the front end portion is liable to be thermally deformed and in order to prevent the deformation, there poses a problem that a temperature of the die can be heated only to about 80°C which is a comparatively low temperature and a long working time period of 2 through 3 hours is needed.

Further, according to the related art method, a divided mark of the die appears on a surface of the thermoplastic tube after having been worked, and therefore, there poses a problem that an outlook thereof is not smooth. Therefore, the insertion assisting piece fabricated by the related art method poses a problem that a friction thereof with the body wall is increased when inserted into the body.

The invention has been carried out in view of such a situation and it is an object thereof to provide a method of working an end portion of a tube for a medical instrument capable of easily worked in a short period of time and capable of providing an end portion having a smooth outer surface.

### SUMMARY

[1] In order to achieve the above-described object, according to an aspect of the invention, A method of working an end portion of a tube for a medical instrument including a thermoplastic tube, a tube end portion of the thermoplastic tube which is formed into a narrowed shape. The method includes: inserting a core member into the tube for the medical instrument and arranging the core member at the end portion of the tube for the medical instrument; covering, with a thermally contracting tube, the end portion of the tube for the medical instrument in which the core member is inserted; and contracting the thermally contracting tube by applying heat at a position of the end portion of the tube for the medical instrument to thereby deform the end portion of the tube for the medical instrument by a contracting force of the thermally contracting tube and the heat from the thermally contracting tube.
   According to [1], the end portion of the tube for the medical instrument is contracted by the contacting force of the thermally contracting tube and the heat from the thermally contracting tube, and therefore, the heat is difficult to transfer to a portion of the tube for the medical instrument other than the end portion, and only the end portion of the tube can be worked firmly in a short period of time. Further, according to [1], the contracted thermally contracting tube is brought into contact with the end portion of the tube for the medical instrument and an outer surface thereof becomes smooth. Therefore, a friction resistance of the outer surface of the end portion of the tube for the medical instrument is reduced and a friction with the body wall is reduced when used as the medical instrument.
[2] According to the method of [1], the core member may be formed by a shape of a cylinder having a hollow portion.
   According to [2], the core member is formed of the cylindrical shape, and therefore, the heat of the core member can be escaped from the hollow portion. Therefore, the heat from the thermally contracting tube can be restrained from being conducted to other than the end portion of the tube for the medical instrument through the core member.
[3] According to the method any of [1] or [2], the core member includes a centering portion and a narrowed portion. The centering portion has an outer diameter substantially the same as an inner diameter of the tube for the medical instrument. The narrowed portion has an outer diameter smaller than the outer diameter of the centering portion. The outer diameter of the narrowed portion is substantially the same as an inner diameter of the deformed end portion of the tube for the medical instrument in the contracting step. And the method any of [1] or [2], may further includes positioning the centering portion by inserting to the tube for the medical instrument.
   According to [3], by inserting the centering portion to the tube for the medical instrument, the core member can be arranged at a center of the tube for the medical instrument.
[4] According to the method any of [1], [2] or [3], The method may further includes forming an projected portion from the core member over periphery of an outer peripheral face of the core member; and arranging the projected streak portion at the end portion of the tube for the medical instrument.
   Accordingto [4], by arranging the projected streak portion at the position of the end portion of the tube for the medical instrument and contracting the portion of the tube for the medical instrument, an end face of the tube for the medical instrument is formed by following the projected streak portion. Therefore, the shape of the end face of the tube for the medical instrument can accurately be worked.
[5] According to the method of [5], the method may further includes: heating the thermally contracting tube by impinging hot wind onto an outer peripheral face of the thermally contracting tube. The hot wind is impinged skewedly by being directed to a side of the end portion from a side of the center portion of the tube for the medical instrument.
   According to [5], the hot wind (that is, heated air) is impinged skewedly from the side of the center portion of the tube for the medical instrument, and therefore, the heat can be restrained from being conducted to other than the end portion of the tube for the medical instrument.
[6] In order to achieve the above-described object, according to another aspect of the invention, the method of working an end portion of a tube for a medical instrument including a thermoplastic tube formed with a pipe line in an axial direction at inside of a tube peripheral wall. The pipe line is closed by narrowing the end portion of the tube. The method includes inserting the core member to the tube for the medical instrument and arranging the core member at an end portion of the tube for the medical instrument; covering, with a thermally contracting tube, the end portion of the tube for the medical instrument in which the core member is inserted; and contracting the thermally contracting tube by applying heat at a position of the end portion of the tube for the medical instrument to thereby (i) deform the end portion of the tube for the medical instrument and (ii) close the end portion of the pipe line by a contracting force of the thermally contracting tube and the heat from the thermally contracting tube.

According to [6], simultaneously with contracting the end portion of the tube for the medical instrument by the contracting force of the thermally contracting tube and the heat from the thermally contracting tube, the end portion of the pipe line can be closed.

According to any of [1], [2], [3], [4], [5] or [6], the end portion of the tube for the medical instrument is contracted by the contracting force of the thermally contracting tube and the heat from the thermally contracting tube, and therefore, only the end portion can be worked firmly in a short period of time and the smooth front end of the outer surface can be provided.

### BRIEF DESCRIPTION OF THE DRAWINGS

Fig. 1 is a perspective view showing an insertion assisting piece fabricated by using the invention.
Fig. 2 is a sectional view showing a front end of an insertion assisting piece in a state of being inserted with an inserting portion of an endoscope.
Fig.3 is a perspective view showing a front end and a core member of a tube and a thermally contracting tube before being worked.
Figs.4A, 4B, 4C and 4D illustrate explanatory views for explaining a procedure of working a front end of a tube.
Fig. 5 is a side sectional view showing a core member having other shape.
Fig.6A and 6B illustrate explanatory views showing a working method of a background art.
Fig. 7 is a perspective view showing an insertion assisting piece of a double balloon type endoscope.
Fig.8 is a sectional view of a front end portion of the insertion assisting piece of Fig.7.
Fig.9 is a perspective view showing a method of working the insertion assisting piece of Fig.7.

### DETAILED DESCRIPTION

A detailed description will be given of preferable embodiments of a method of working an end portion of a tube for a medical instrument according to the invention in reference to the drawings as follows.

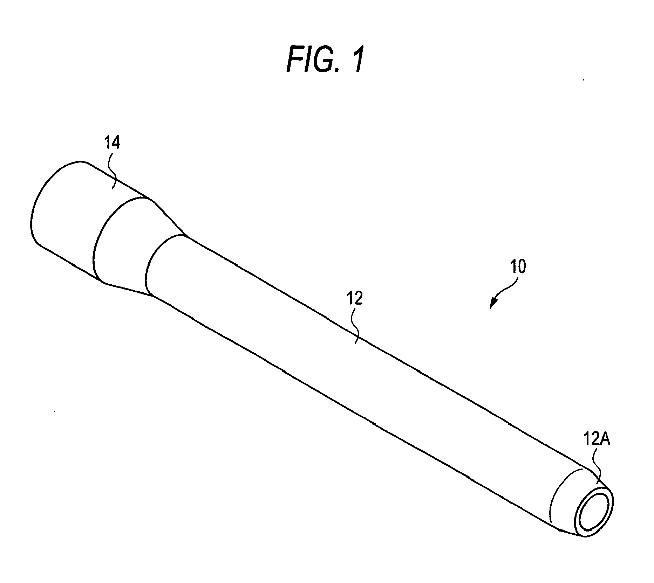

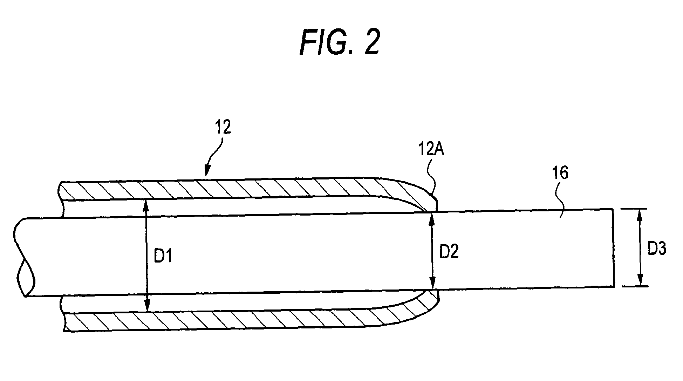

Fig. 1 is a perspective view showing an insertion assisting piece fabricated by using a method of working an end portion of a tube for a medical instrument according to the invention. Further, Fig.2 is a sectional view showing a front end portion of an insertion assisting piece in a state of being inserted with an inserting portion of an endoscope.

As shown in Fig. 1, an insertion assisting piece 10 includes a thermoplastic tube having a flexibility (hereinafter, tube) 12 for a medical instrument and a hard grabbing portion 14 provided at a base end of the tube 12. The grabbing portion 14 is formed by a cylindrical shape by a resin material and the base end of the tube 12 is fixed to the grabbing portion 14. The insertion assisting piece 10 is operated by grabbing the grabbing portion 14 by an operator.

The tube 12 is formed by a cylindrical shape by a thermoplastic resin material of polyurethane or the like and the tube 12 is inserted with an inserting portion 16 (refer to Fig.2) of an endoscope. The inner diameter D1 is formed to be larger than an outer diameter D3 of the inserting portion 16 of the endoscope by 0.5 through 2mm on one side thereof to be able to insert and detach the inserting portion 16 of the endoscope smoothly to and from the tube 12.

The tube 12 has the converging shape of converging a diameter of a front end 12A thereof. An inner diameter D2 of the front end 12A is formed by a dimension substantially equal to the outer diameter D3 of the inserting portion 16 of the endoscope. Therefore, when the inserting portion 16 is inserted into the tube 12, a gap is hardly produced between an outer peripheral face of the inserting portion 16 and the front end 12A of the tube 12. Therefore, when the inserting portion 16 of the endoscope is operated to push and pull to and from the insertion assisting piece 10, the body wall (for example, the intestinal wall) can be prevented from being entrapped to the gap between the inserting portion 16 and the insertion assisting piece 10. Further, when a lubricant (for example, water) is supplied to an inner portion of the insertion assisting piece 10, the lubricant between the insertion assisting piece 10 and the inserting portion 16 can be restrained from flowing out from the front end 12A of the insertion assisting piece 10.

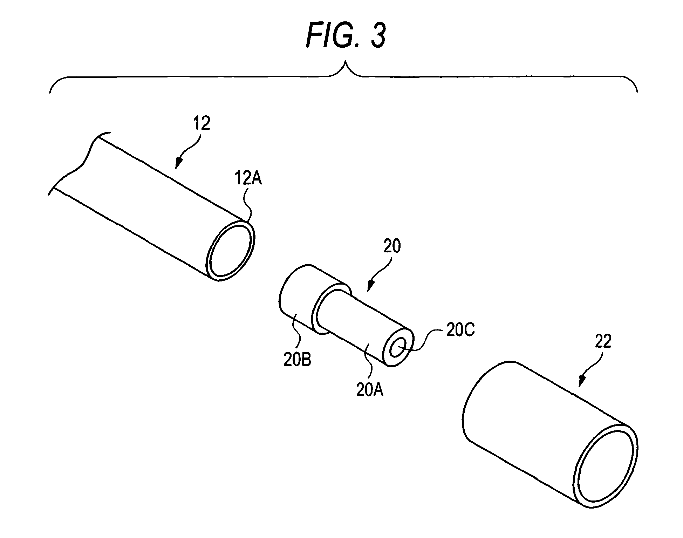

Next, a method of working the front end of the tube 12 will be explained. Fig. 3 is a perspective view showing the front end 12A of the tube before being worked, a core member 20 as a working piece and a thermally contracting tube 22. As shown in the drawing, the tube 12 before being worked is formed by a constant diameter.

The core member 20 is made of a material having a low heat transfer of a resin or the like. Further, the material of the core member 20 may be a heat insulating material. Further, the core member 20 is formed by a cylindrical shape as a whole to be able to radiate heat from a hollow portion 20C thereof. Therefore, a temperature of the core member 20 is made to be difficult to rise even when heat is conducted from the thermally contracting tube 22 mentioned later.

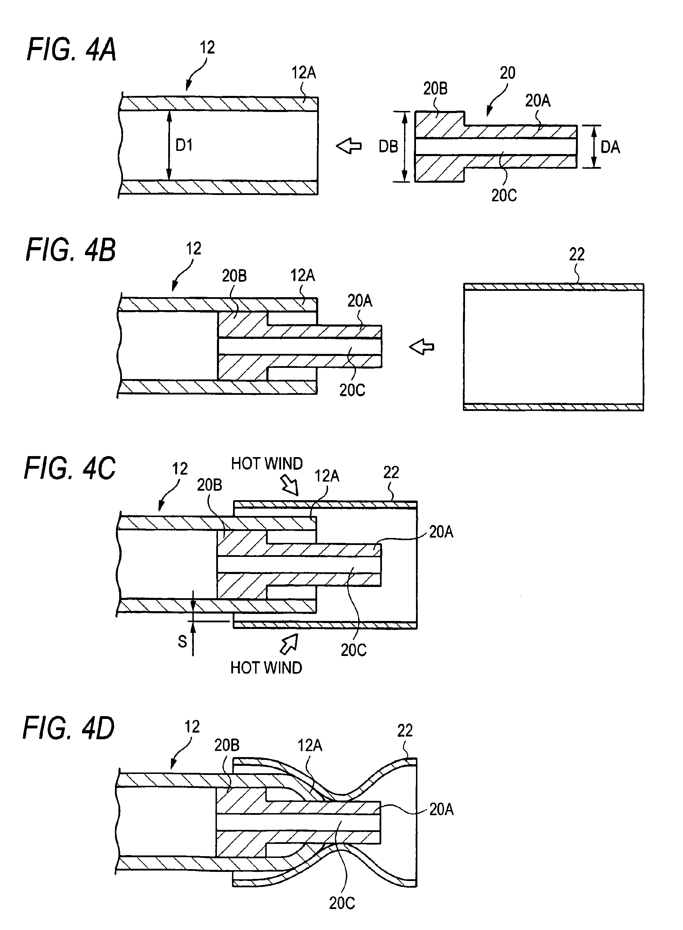

Further, the core member 20 includes a narrowed portion 20A having a small outer diameter and a centering portion 20B having a large diameter provided at an end portion of the narrowed portion 20A. An outer diameter DA of the narrowed portion 20A (refer to Fig.4A) is set to a target inner diameter dimension of working the front end 12A of the tube 12 (that is, D2 of Fig.2). On the other hand, an outer diameter DB of the centering portion 20B (refer to Fig.4A) is formed by dimension substantially the same as the inner diameter D1 of the tube 12. Therefore, when the centering portion 20B is inserted into the tube 12, the centering portion 20B is arranged in a state of overlapping a center axis of the centering portion 20B and the center axis of the tube 12, and the core member 20 is accurately arranged at a center of the tube 12.

On the other hand, the thermally contracting tube 22 is a tube having a property of being contracted when heated and is made of a material of, for example, polyolefin, ethylenepropylene rubber, silicone rubber, FEP or the like. The thermally contracting tube 22 is formed by a shape of a cylinder having a size suitable for covering the tube 12, that is, the inner diameter larger than an outer diameter of the tube 12. It is preferable that the size of the thermally contracting tube 22 is formed such that more or less gap S (refer to Fig.4C) is left relative to the outer peripheral face of the tube 12. Here, it is preferable that the gap S has a size capable of inserting an edge of a scissor or the like, for example, about 3mm on one side.

Fig.4A through Fig.4D are explanatory views for explaining a procedure of working the front end 12A of the tube 12.

First, as shown in Fig.4A, the core member 20 is inserted into the tube 12 from the centering portion 20B. As shown in Fig. 4B, the narrowed portion 20A of the core member 20 is arranged at a position of the front end 12A of the tube 12. Thereby, the core member 20 is arranged on the center axis of the tube 12 by the centering portion 20B.

Next, the thermally contracting tube 22 is covered onto the tube 12. As shown in Fig. 4C, a center portion of the thermally contracting tube 22 is arranged at the position of the front end 12A of the tube 12.

Next, the center portion of the thermally contracting tube 22 is heated. Although a heating method is not particularly limited, for example, hot wind is blown over one periphery of the outer peripheral face of the thermally contracting tube 22 by a drier (not illustrated). At this occasion, it is preferable to blow wind skewedly from a side of the center portion to a side of the front end of the tube 12. Thereby, hot wind impinges on the thermally contracting tube 22 and thereafter flows to an outer side (right side of Figs. 4C), and therefore, the side of the center portion of the thermally contracting tube 22 can be prevented from being thermally contracted.

The heated thermally contracting tube 22 is contracted as shown in Fig.4D. Thereby, the front end 12A of the tube 12 is pressed from an outer side by the thermally contracting tube 22, heat of the thermally contracting tube 22 is conducted to the front end 12A of the tube 12, and therefore, the diameter is contracted by thermal deformation. The contracted tube 12 is brought into contact with the outer peripheral face of the narrowed portion 20A of the core member 20 to determine a shape thereof.

After sufficiently contacting the thermally contracting tube 22, a heating operation is stopped. Successively, the thermally contracting tube 22 is broken. At this occasion, more or less gap S is present between the thermally contracting tube 22 and the tube 12, and therefore, the thermally contracting tube 22 can be broken by inserting an edge of a scissor or the like.

Successively, the core member 20 is drawn from the tube 12. At this occasion, the core member 20 may be drawn from the base end side of the tube 12 or may be drawn from the front end side while elastically deforming the front end 12A. By the above-described operation, the tube 12 is fabricated with the front end 12A which is narrowed.

The front end 12A of the tube 12 worked as described above is contracted by being pressed by the thermally contracting tube 22, and therefore, an outer surface of the front end 12A after having been worked becomes smooth similar to an inner surface of the thermally contracting tube 22. Therefore, according to the embodiment, the tube 12 having a smooth outer surface of the front end 12A can be provided. The tube 12 having the smooth outer surface of the front end 12A in this way is suitable as a medical instrument since friction thereof with the body wall is small when inserted into the body.

Further, according to the embodiment, heat is transferred by bringing the contracted portion of the thermally contracting tube 22 into contact only with the front end 12A of the tube 12, and therefore, heat is hardly conducted to a portion which is not brought into contact with the thermally contracting tube 22. Therefore, the thermally contracting tube 22 can be heated at a high temperature equal to or higher than 100°C, and the front end 12A of the tube 12 can be worked by a short time period of about 15 minutes.

Further, although according to the above-described embodiment, the core member 20 is formed by the cylindrical shape, the shape of the core member 20 is not limited thereto but may be formed by a shape of a circular column.

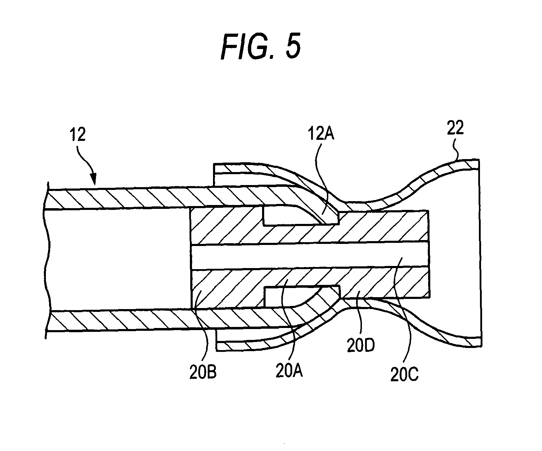

Further, as shown in Fig.5, the core member 20 may be provided with a projected streak portion 20D at the outer peripheral face of the narrowed portion 20A. The projected streak portion 20D of Fig. 5 is formed over the periphery of the outer peripheral face of the narrowed portion 20A. When worked by using the core member 20, the projected streak portion 20D is arranged on an immediate outer side of the front end 12A of the tube 12. Thereby, when the thermally contracting tube 22 is contracted, an amount of elongating the front end 12A of the tube 12 in a longitudinal direction is restrained by the projected streak portion 20D, and therefore, the front end 12A of the tube 12 can accurately be worked.

Further, in the above-described embodiment, a constitution or use of the insertion assisting piece 10 is not particularly limited but, for example, the insertion assisting piece 10 may be used by mounting a balloon (not illustrated) at an outer peripheral face of a front end of the tube 12. Further, the insertion assisting piece 10 attached with a balloon may be used as a double balloon type endoscope apparatus along with the endoscope mounted with a balloon (not illustrated) at the outer peripheral face of the front end of the inserting portion 16. Particularly, in the case of the double balloon type endoscope apparatus, the inserting portion 16 of the endoscope is repeatedly operated to insert and detach to and from the insertion assisting piece 10, and therefore, an accuracy of working of the front end 12A of the insertion assisting piece 10 is important and it is preferable to apply the invention thereto.

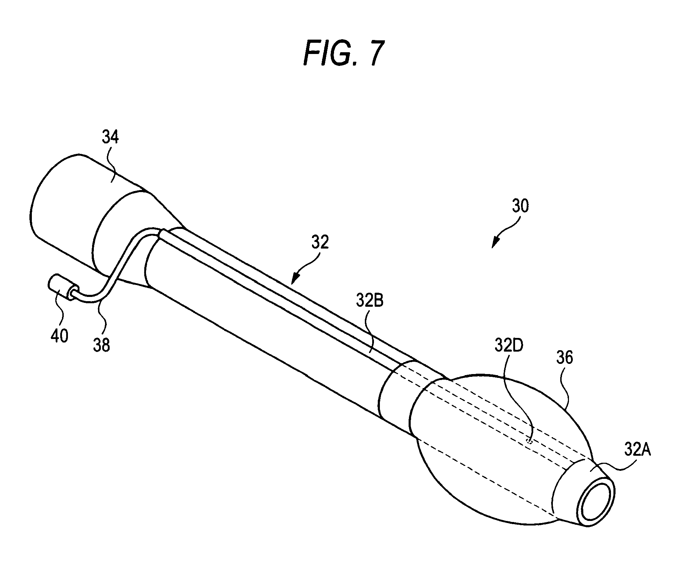

Next, an explanation will be given of a method of working an insertion assisting piece 30 used in a double balloon type endoscope in reference to Fig.7 through Fig.9. As shown in the drawings, the double balloon type insertion assisting piece 30 includes a thermoplastic tube 32 having a flexibility, a grabbing portion 34 provided at a base end of the tube 32, and a balloon 36 attached to an outer peripheral face of the front end of the tube 32. The grabbing portion 34 is formed by a cylindrical shape by a resin material and the grabbing portion 34 is fixed with the base end of the tube 32.

The tube 32 is formed in a cylindrical shape by a thermoplastic resin material of polyurethane or the like. A peripheral wall of the tube 32 includes a thick-walled portion 32B along an axial direction, and a pipe line 32C is formed at an inner portion of the thick-walled portion 32B along the axial direction. A base end of the pipe line 32C is connected with an air blowing tube 38 and an air blowing port 40 is provided at a front end of the air blowing tube 38. The air blowing port 40 is connected to a balloon control apparatus, not illustrated, and air is supplied and sucked by the balloon control apparatus.

An outer peripheral face of the tube 32 is formed with a vent hole 32D at a position of an inner side of the balloon 36 and the vent hole 32D is communicated with the pipe line 32C. Therefore, by supplying and sucking air to and from the air blowing port 40, air is supplied and sucked to and from the inner portion of the balloon 36 and the balloon 36 is expanded and contracted.

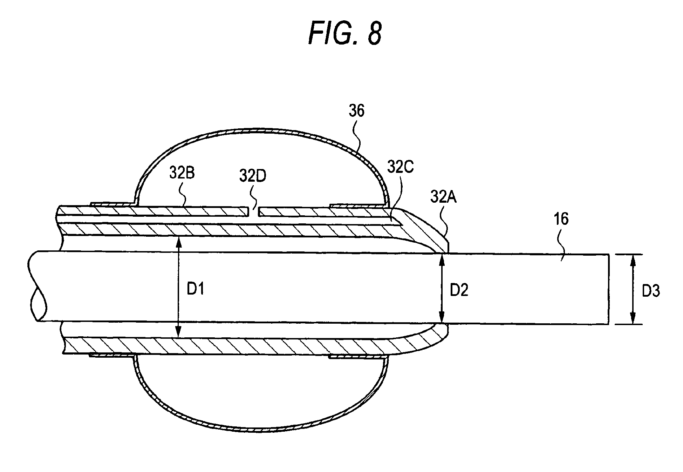

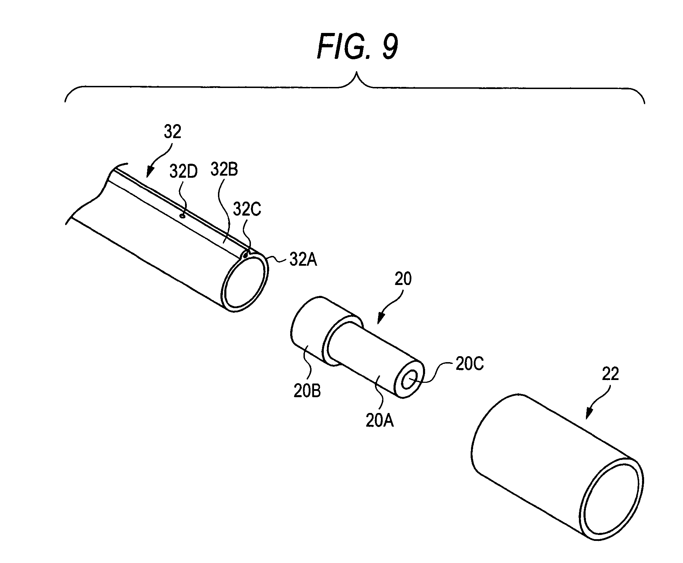

Meanwhile, as shown in Fig.9, all of sections of the tube 32 before being worked orthogonal to an axis thereof are formed by the same shape. Therefore, a front end of the pipe line 32C is communicated to an end face of the tube 32. However, air is leaked from the front end of the pipe line 32C from the front end of the pipe line 32C when the end face is as it is and the balloon 36 cannot be expanded and contracted, and therefore, as shown in Fig.8, it is necessary to close the front end of the pipe line 32C.

Hence, according to the embodiment, the front end of the pipe line 32C is worked to close simultaneously with narrowing the tube 32. The tube 32 can be worked by the method explained in reference to Fig. 4A through Fig. 4D. That is, the core member 20 is inserted to the tube 12 from the centering portion 20B, and the narrowed portion 20A of the core member 20 is arranged at a position of the front end 32A of the tube 32. Successively, the thermally contracting tube 22 is covered onto the tube 32 and the center portion is arranged at a position of the front end 32A of the tube 32. Next, the center portion of the thermally contracting tube 22 is heated to be contracted. Thereby, the front end 32A of the tube 32 is pressed from the outer side by the thermally contracting tube 22, heat of the thermally contracting tube 22 is conducted to the front end 32A of the tube 32, and therefore, the front end 32A is contracted by thermal deformation. Simultaneously therewith, a peripheral portion of the front end of the pipe line 32C is thermally deformed to close the front end of the pipe line 32C. After sufficiently contracting the thermally contracting tube 22, the heating operation is stopped and the thermally contracting tube 22 is broken. Successively, the core member 20 is drawn from the tube 32. By the above-described operation, the front end of the pipe line 32C is closed and the tube 32 is fabricated with the front end 32A which is narrowed.

By using the working method of the invention in this way, the front end of the tube 32 can be narrowed and the front end of the pipe line 32C can be worked to close simultaneously.

Further, although according to the above-described embodiment, an explanation has been given by the example of working the insertion assisting pieces 10, 30, a tube for a medical instrument to be worked is not limited thereto but, for example, applicable to working a front end of a tube of injecting a medical solution into the body or the like.

## Claims

1. A method of working an end portion of a tube for a medical instrument comprising a thermoplastic tube, a tube end portion of the thermoplastic tube which is formed into a narrowed shape, the method comprising:
inserting a core member into the tube for the medical instrument and arranging the core member at the end portion of the tube for the medical instrument;
covering, with a thermally contracting tube, the end portion of the tube for the medical instrument in which the core member is inserted; and
contracting the thermally contracting tube by applying heat at a position of the end portion of the tube for the medical instrument to thereby deform the end portion of the tube for the medical instrument by a contracting force of the thermally contracting tube and the heat from the thermally contracting tube.

2. The method of working the end portion of the tube for the medical instrument according to claim 1,
wherein the core member is formed of a shape of a cylinder having a hollow portion.

3. The method of working the end portion of the tube for the medical instrument according to claim 1 or 2,
wherein the core member comprises:
a centering portion having an outer diameter substantially the same as an inner diameter of the tube for the medical instrument; and
a narrowed portion having an outer diameter smaller than the outer diameter of the centering portion, the outer diameter of the narrowed portion which is substantially the same as an inner diameter of the deformed end portion of the tube for the medical instrument in the contracting step, and
the method further comprising:
positioning the centering portion by inserting to the tube for the medical instrument.

4. The method of working the end portion of the tube for the medical instrument according to any one of claims 1 through 3, further comprising:
forming an projected portion from the core member over periphery of an outer peripheral face of the core member; and
arranging the projected streak portion at the end portion of the tube for the medical instrument.

5. The method of working the end portion of the tube for the medical instrument according to any one of claims 1 through 4, further comprising:
heating the thermally contracting tube by impinging hot wind onto an outer peripheral face of the thermally contracting tube,
wherein the hot wind is impinged skewedly by being directed to a side of the end portion from a side of the center portion of the tube for the medical instrument.

6. A method of working an end portion of a tube for a medical instrument comprising a thermoplastic tube formed with a pipe line in an axial direction at inside of a tube peripheral wall, the pipe line which is closed by narrowing the end portion of the tube, the process comprising;
inserting the core member to the tube for the medical instrument and arranging the core member at an end portion of the tube for the medical instrument;
covering, with a thermally contracting tube, the end portion of the tube for the medical instrument in which the core member is inserted; and
contracting the thermally contracting tube by applying heat at a position of the end portion of the tube for the medical instrument to thereby (i) deform the end portion of the tube for the medical instrument and (ii) close the end portion of the pipe line by a contracting force of the thermally contracting tube and the heat from the thermally contracting tube.
